Europäisches Patentamt

European Patent Office (11) Publication number: **0 077 159**
**A1**

Office européen des brevets

(12) **EUROPEAN PATENT APPLICATION**

(21) Application number: **82305253.5**

(51) Int. Cl.³: **A 61 B 17/00,** A 61 M 29/00

(22) Date of filing: **04.10.82**

(30) Priority: **14.10.81 GB 8130980**

(43) Date of publication of application: **20.04.83**
Bulletin 83/16

(84) Designated Contracting States: **AT BE CH DE FR GB IT LI LU NL SE**

(71) Applicant: **Atkins, Brian Norman, Brian Atkins Engineering (Surgical) Trinity Street, Leamington Spa Warwickshire (GB)**
Applicant: **Whatmore, William John, The Barn, Stoneleigh Warwickshire (GB)**

(72) Inventor: **Atkins, Brian Norman, Brian Atkins Engineering (Surgical) Trinity Street, Leamington Spa Warwickshire (GB)**
Inventor: **Whatmore, William John, The Barn, Stoneleigh Warwickshire (GB)**

(74) Representative: **Anderson, John Robert Browning et al, WALFORD & HARDMAN BROWN Trinity House Hales Street, Coventry, CV1 1NP (GB)**

(54) **Vertebrae spreader.**

(57) A vertebrae spreader for use in performing the anterior fusion of cervical vertebrae by inserting a bone graft dowel between a pair of vertebrae. The vertebrae spreader is in the form of a tubular sleeve (11) having at one end thereof a pair of substantially semi-cylindrical end portions (12, 13) formed separately from the remainder of the sleeve (11) and each mounted on a respective springy blade (14) extending longitudinally of the cylindrical wall of the sleeve (11) and resiliently biased towards each other into circumferentially contracted positions. Adjustable expander rods (19) are movable into and out of positions between the end portions (12, 13) in which the expander rods (19) will force the end portions (12, 13) apart laterally of the sleeve (11) from their circumferentially contracted positions against the resilience of the blades (14). Each end portion (12, 13) has at least a pair of pins (16) extending longitudinally-outwardly therefrom and locatable in respective locating holes which have previously been made in a peripheral wall of each of the pair of vertebrae (1, 2).

# 0077159

## VERTEBRAE SPREADER

The invention relates to a vertebrae spreader and is particularly, but not exclusively, concerned with a vertebrae spreader for spreading apart two adjacent cervical vertebrae in an operation to fuse the two vertebrae together by the insertion therebwteen of a bone graft.

The anterior fusion of cervical vertebrae by means of a bone graft has been developed by R.B.Cloward. In the Cloward method two adjacent cervical vertebrae are prepared to receive the bone graft by using a tubular drill guide which at its forward end has four forwardly-projecting pins. The drill guide is inserted through an incision in the front of the neck and two of the pins are driven into one of the vertebrae and the other two pins are driven into the other vertebra. A drill is then passed through the drill guide and a blind hole of predetermined depth is drilled to straddle the two vertebrae and the intervertebral space from which the disc or loose disc material has previously been cleared. Thus part of the hole is formed in each of the vertebrae. The drill and the drill guide are then removed. A bone graft in the form of a dowel which is a tight fit in the hole is prepared and this has to be driven into the hole. In order to do this the two vertebrae have to be spread apart temporarily. This spreading operation is performed in the Cloward method by using a spreading tool similar to expanding forceps of which the jaws are inserted between the vertebrae and engaged with an upper face of the lower vertebra and an opposite lower face of the upper vertebra or by otherwise forcing the two vertebrae apart by a tool engaged between the opposite upper and lower faces of the vertebrae. This way of spreading the vertebrae incurs the risk of damaging the vertebrae. An object of the invention is to provide a vertebrae spreader which does not act between the opposite upper and lower faces of the vertebrae and so reduces the risk of damaging the vertebrae.

According to the invention, the vertebrae spreader comprises a tubular sleeve having at one end thereof a pair of substantially semi-cylindrical end portions formed separately from the remainder of the sleeve and each mounted on a respective springy blade extending longitudinally of the cylindrical wall of the sleeve and resiliently biased towards each other into circumferentially contracted positions by the resilience of the blades; adjustable expander means movable into and out of positions between said end portions in which the expander means will force said end portions apart laterally of the sleeve from their circumferentially contracted positions against the resilience of the blades and on each said end portion at least a pair of pins extending longitudinally-outwardly therefrom and locatable in respective locating holes which have previously been made in a peripheral wall of each of a pair of vertebrae to be spread apart in their longitudinal direction, the sleeve having a longitudinal throughway of a diameter through which a bone graft dowel is freely insertable.

The blades may be formed by parts of the cylindrical wall of the sleeve in the manner of a spring collet.

The expander means may conveniently be rods adjustable longitudinally of the sleeve and having ends insertable between said end portions of the sleeve. The ends of the rods which are insertable between said end portions may be of non-circular cross-section or may be tapered in a direction to move said end portions apart when said rods are inserted between said end portions. The rods may be adjustable longitudinally of the sleeve by means of screw-threaded portions of the rods extending through respective screw-threaded guides on the sleeve.

The vertebrae spreader according to any of the three immediately preceding paragraphs may be used in conjunction with a separate tubular drill guide to be used to receive and guide a drill while the latter is being used to drill a hole to straddle a pair of adjacent vertebrae, as aforesaid, the drill guide having a plurality of pins extending longitudinally from the forward end thereof and which are driven into the peripheral wall of each of the vertebrae to locate the drill guide prior to drilling, the pins on the drill guide producing at least two locating holes in each vertebrae, which locating holes are subsequently used, after removal of the drill guide, to locate the respective end portions of the vertebrae spreader, the vertebrae spreader and the drill guide each having the same number of pins arranged in the same configuration to enable the spreader, when the end portions thereof are in their contracted positions, to be located by the pins thereon in the same position as was the drill guide with respect to the vertebrae.

The vertebrae spreader, as aforesaid, may also be used in conjunction with a cylindrical mandrel therefor which has a diameter substantially equal to that of the drill to be used to drill the hole which straddles the vertebrae, whereby the mandrel is insertable in the hole after the drill and the drill guide have been removed and thus forms a support along which the vertebrae spreader is slidable to enable the pins of the latter to be located by and driven into the locating holes previously made in the vertebrae by the pins of the drill guide, the diameter of the mandrel being smaller than the diameter of the throughway in the spreader.

Instead of using a separate drill guide, the vertebrae spreader may also be used as a drill guide by providing on the vertebrae spreader a locking sleeve which is movable between a first position in which said pair of semi-cylindrical end portions are held by the locking sleeve in positions in which they are contracted, thereby enabling the vertebrae spreader to be used as said drill guide, and a second position in which said pair of semi-cylindrical end portions are free to be moved to their circumferentially expanded positions by said expander means. The locking sleeve may be held in said second position by manually-releasable catch means.

The vertebrae spreader, as aforesaid, may also be used in conjunction with a tool for inserting the bone graft dowel through the throughway in the spreader and into the hole which straddles the vertebrae after the latter have been spread apart by appropriate adjustment of the expander means of the spreader to force said end portions thereof and hence the vertebrae apart, the inserting tool having a cylindrical body which is insertable into and slidable in the throughway of the spreader and which has a pin extending forwardly therefrom on which the bone graft dowel is supported as a substantially co-axial forward extension of the body of the inserting tool. The inserting tool may have an adjustable stop thereon which is locatable against the outer end of the vertebrae spreader to limit the penetration distance of the bone graft dowel into the hole which straddles the vertebrae before the latter are closed together onto the dowel by adjustment of the adjusting means of the vertebrae spreader to permit the end portions thereof to return to their circumferentially contracted positions.

The invention also provides a set of instruments or tools comprising the aforesaid vertebrae spreader and separate drill guide or the aforesaid combined vertebrae spreader and drill guide, together with the aforesaid bone graft dowel inserting tool.

By way of example two forms of a vertebrae spreader in accordance with the invention and associated tools and instruments all for use in performing a fusion of two adjacent cervical vertebrae by the insertion therebetween of a bone graft and the method of use are now described with reference to the accompanying drawings, in which:-

Figure 1 is a perspective sketch of the two vertebrae and shows a drill guide in position ready for drilling of the hole to straddle the two vertebrae;

Figure 2 is a similar sketch showing the drill in position in the drill guide shown in Figure 1;

Figure 3 is a perspective sketch of the two vertebrae showing a mandrel for the vertebrae spreader in position in the hole which has been drilled to straddle the two vertebrae;

Figure 4 is a similar sketch showing the first form of vertebrae spreader mounted on the mandrel shown in Figure 3;

Figure 5 is a side elevation of the vertebrae showing the vertebrae spreader shown in Figure 4 after the mandrel has been removed and in the position in which the vertebrae have been spread apart in their longitudinal direction;

Figure 6 is an end view of the vertebrae spreader in the direction of arrows VI in Figure 5;

Figure 7 is a plan view in the direction of arrow VII in Figure 5;

Figure 8 is a perspective view of the bone graft dowel inserting tool to be used in conjunction with the vertebrae spreader and the dowel itself;

Figure 9 is a front view of the two vertebrae showing the bone graft dowel after insertion into the hole which straddles the vertebrae;

Figure 10 shows the second form of vertebrae spreader in accordance with the invention and which is also used as a drill guide after being located on the two vertebrae;

Figure 11 shows the combined vertebrae spreader and drill guide shown in Figure 10 being used as a drill guide;

Figure 12 shows the combined vertebrae spreader and drill guide shown in Figures 10 and 11 in its contracted position;

Figure 13 shows the combined vertebrae spreader and drill guide shown in Figures 10-12 in an expanded position and locating the bone graft dowel inserting tool of Figure 8, and

Figure 14 shows the bone graft dowel after insertion into the hole which straddles the vertebrae and the combined vertebrae spreader and drill guide of Figures 10-13 being removed.

The two forms of vertebrae spreader and the associated tools and instruments now to be described with reference to the accompanying drawings enable the operation of fusion of two cervical vertebrae by the insertion therebetween of a bone graft dowel to be performed by the known Cloward method modified by the step of spreading apart the vertebrae by using the vertebrae spreader provided by this invention instead of spreading the vertebrae by using a spreader similar to forceps or some other tool acting between a lower surface of the upper of the two vertebrae and an opposite upper surface of the lower of the two
vertebrae. This is an important difference because
as will be evident from the following description,
each of the vertebrae spreaders provided by this
invention enables the vertebrae to be more safely
spread apart in a precise manner and reduces the
risk of damage to the vertebrae. Furthermore each
of the vertebrae spreaders enables a surgeon of
lesser skill or experience to perform the operation
safely.

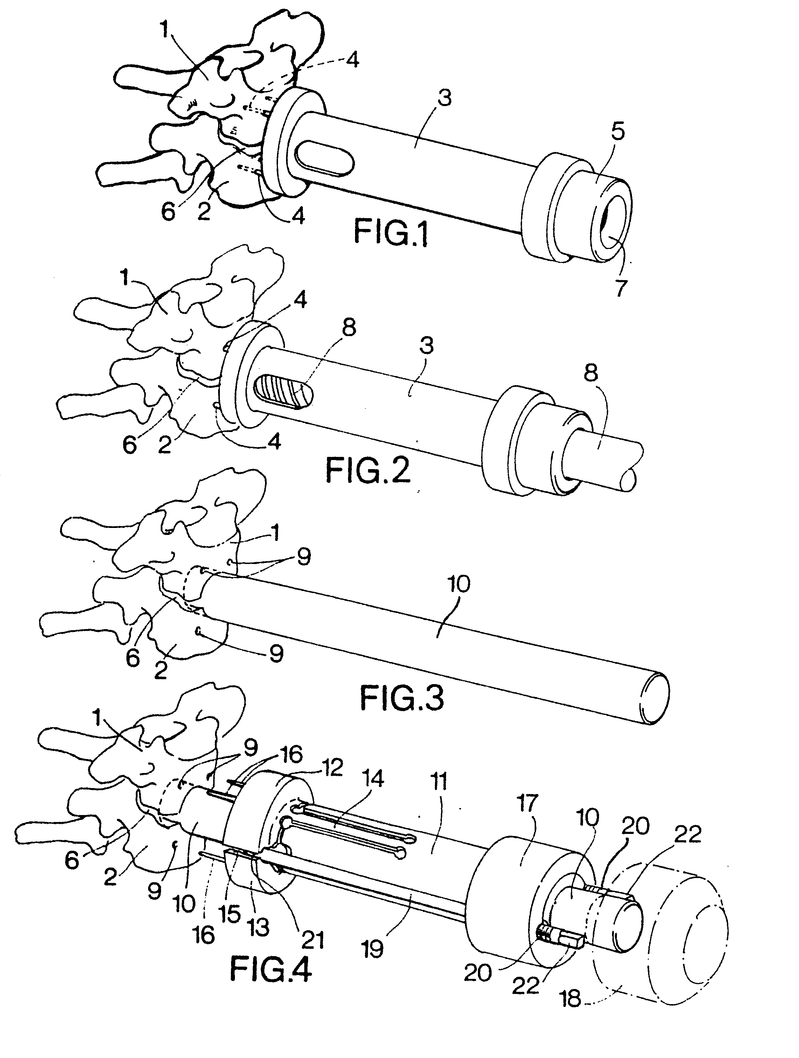

The operation will now be described with reference to
Figures 1-9, that is with reference to the first form
of the vertebrae spreader.

The patient is prepared for the operation by placing
the patient in a supine position and by making an
incision in the front of the neck to expose the
two cervical vertebrae to be fused. These two
vertebrae are shown at 1 and 2 in Figure 1 and some
subsequent Figures. The first tool to be used is
the drill guide 3. This is similar to that used in
the Cloward method in that it comprises a tubular
sleeve into which the drill is to be inserted and
also has a plurality of longitudinally-extending pins
4 which are driven into the front walls of the
vertebrae by striking the outer end 5 of the drill
guide by a hammer. However, the spacing and
configuration of the pins 4 differ from those in the
drill guide, as used in the Cloward method, so as to
enable the vertebrae spreader to be more easily
accommodated on the vertebrae. There are four pins
4 arranged in two pairs, one pair of pins is driven
into one of the two vertebrae and the other pair of pins is driven into the other of the two vertebrae. The length of the pins 4 is such that the pins will satisfactorily locate the drill guide 3 but will not penetrate the vertebrae and so no harm will be done to the spinal cord. Before the pins 4 of the drill guide 3 are driven into the vertebrae, the intervertebral space 6 is cleared of disc material or the disc is removed. A locating mandrel (not shown) may be used to locate the drill guide 3, the locating mandrel having a flattened or reduced end which is fitted between the two vertebrae. The locating mandrel is removed through the drill guide when the latter has been properly located on the two vertebrae.

The next stage is the insertion of the drill through the axial throughway 7 in the drill guide 3. The purpose of drilling is to drill a hole which straddles the two vertebrae, i.e. part of the hole is formed in the upper vertebra 1 and part is formed in the lower vertebra 2. The diameter of the throughway 7 is substantially equal to that of the drill 8, shown in Figure 2, and is a little smaller than the diameter of the bone graft dowel which is to be fitted into the hole. As in the known Cloward method, the permitted inward travel of the drill 8 is accurately predetermined by a stop on the drill engaging the outer end of the drill guide, thereby to limit the depth of the hole and to prevent the drill from penetrating to the interior of the vertebrae and into the spinal cord.

When the hole has been drilled, the drill 8 and the drill guide 3 are removed, thereby to expose the hole for receiving the bone graft dowel and the four locating holes made in the vertebrae by the pins 4. These locating holes are indicated at 9 in Figure 3.

In accordance with this invention, a cylindrical mandrel 10 is inserted into the hole which has been drilled to straddle the vertebrae 1, 2 as shown in Figure 3. The mandrel 10 has a diameter which makes it a tight fit in the hole. The purpose of the mandrel 10 is to support the vertebrae spreader which is shown in Figure 4. This comprises a cylindrical tube 11 having an axial throughway of diameter slightly greater than that of the mandrel 10, as it has to permit the insertion therethrough of the bone graft dowel, as herineafter explained. The forward end of the vertebrae spreader is formed by two substantially semi-cylindrical collar portions 12, 13 which are carried on respective springy blades 14 extending along part of the length of the tube 11 and formed by cutting longitudinal slots in the cylindrical wall of the tube 11. Alternatively the collar portions 12, 13 may be supported on springy blades secured to the outside of the tube 11. The blades 14 resiliently bias the collar portions 12, 13 inwardly of the tube 11 so that the collar portions 12, 13 are diametrically contracted together to define a cylindrical collar of a first minimum diameter, but with diametrically-opposed gaps 15, of which only one is shown in Figure 4, therebetween. Each of the collar portions 12, 13 carries a pair of forwardly-longitudinally extending locating pins 16. The spacing and configuration of these pins 16 are the same as those of the pins 4 on the drill guide 3, when the collar portions 12, 13 are contracted together and so when the vertebrae spreader is slid along the mandrel 10 as shown in Figure 4, the pins 16 will locate in respective holes 9, which were formed by the pins 4 on the drill guide 3.    The pins 16 are driven firmly into the holes 9 by engaging an outer end cylindrical flange or fixed collar 17 on the tube 11 by means of a removable anvil 18 and striking the latter with a hammer.

The vertebrae spreader also has two longitudinally-extending rods 19 which have near their outer ends screw-threaded portions 20 engaging screw-threaded holes in the cylindrical flange or collar 17.    The rods 19 extend into the gaps 15 between the collar portions 12, 13 at their inner ends 21, which are tapered.    Thus as the rods 19 are screwed into the respective holes in the cylindrical flange or collar 17 by means of outer end portions 22 to be engaged by a spanner or screw-driver, the collar portions 12, 13 are forced apart by the tapered ends 21 of the rods 19 and conversely when the rods 19 are turned in the reverse direction, the resilient blades 14 will return the collar portions 12, 13 to their contracted positions.

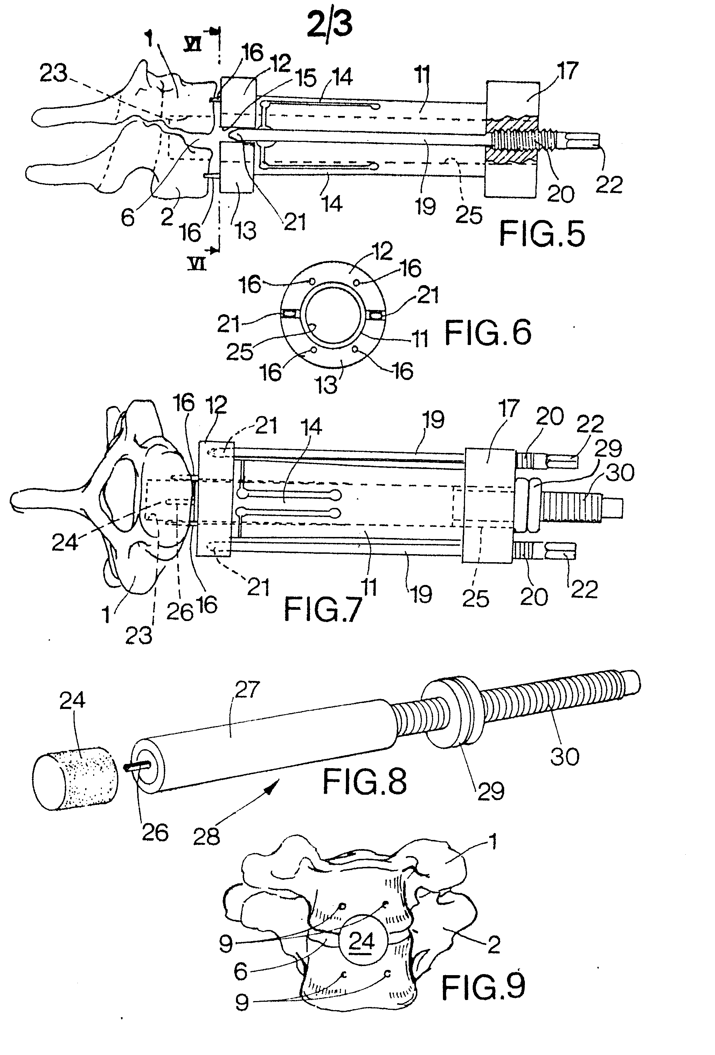

When the vertebrae spreader has been correctly positioned, with the pins 16 located in the holes 9, the collar portions 12, 13 being in their contracted minimum diameter positions, the mandrel 10 is withdrawn from the hole indicated at 23 in Figures 5 and 7 which straddles the vertebrae 1, 2.    Then the rods 19 are screwed in, thereby to circumferentially expand the collar portions 12, 13 to the positions shown in Figure 5.    As the pins 16 are located in the respective locating holes 9 in the vertebrae 1, 2, the latter will be spread apart, thereby increasing the size of the intervertebral gap 6 and also increasing the diameter of the hole 23 slightly to be able to receive the bone-graft dowel. This method of spreading the vertebrae 1, 2 is safer and more precise, as the turning of the rods 19 can be precisely performed, than the known method of prising apart the vertebrae by using expanding forceps, or some other tool, which engages between the vertebrae instead of locating in the front walls thereof as does the vertebrae spreader provided by this invention.

The tube 11 of the vertebrae spreader has a central throughway 25 of diameter which will permit the passage therethrough of the bone-graft dowel, which is of larger diameter than the hole 23 before expansion of the latter by the spreading of the vertebrae 1, 2. The dowel 24 which is shown in Figures 7-9 is prepared to a precise length and required diameter from a portion of bone removed from another bone of the patient, for example, the ilium, or obtained from a bone bank. For example, the dowel may be made of ox bone or kiel bone, obtained from a calf. Alternatively the dowel may be made of a synthetic material.

The dowel 24 has a blind central hole in one end thereof by which it is located on a pin 26 extending from a cylindrical portion 27 of an inserting tool 28 shown in Figures 7 and 8. The pin 26 may be split to enable it to be splayed apart slightly to provide a tight mounting for the dowel. The dowel 24 thus forms a removable forward cylindrical extension of the portion 27.   The dowel 24 and the portion 27 of the inserting tool 28 are pushed into the throughway 25 of the tube 11 until the dowel 24 has been inserted into the hole 23 and the outer end of the dowel 24 is substantially flush with the front of the two vertebrae 1, 2.   The depth of penetration of the dowel 24 into the hole 23 is controlled by a stop 29 which has been pre-adjusted on a screw-threaded portion 30 of the inserting tool 28.   The tube 11, being correctly aligned co-axially with the hole 23, also ensures that the dowel 24, which is guided by the tube 11, will be co-axially aligned with the hole 23 and thus be positioned in the correct position.

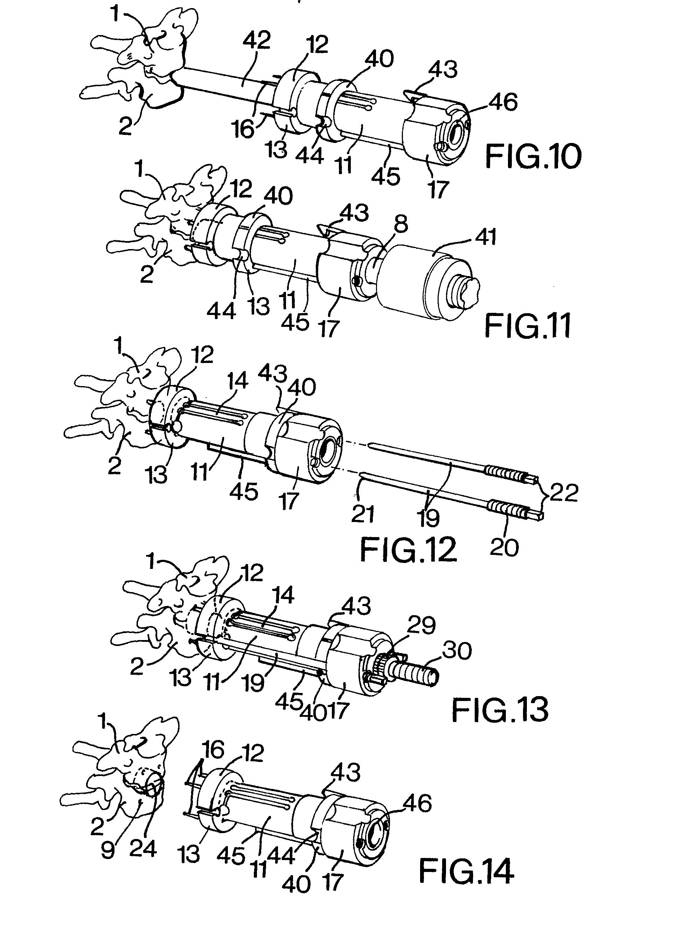

The rods 19 are then screwed in the outward direction, thereby to permit the collar portions 12, 13 to be contracted towards each other to their minimum diameter position by the resilient blades 14.   This causes the two vertebrae 1, 2 to be moved together to grip the dowel 24 in the hole 23.   Then the inserting tool 28 and the vertebrae spreader are withdrawn leaving the dowel 24 in position as shown in Figure 9.

The operation is then finished as in the known Cloward method and in the course of time, the vertebrae 1 and 2 and the bone graft dowel 24 will fuse together.

Several certical vertebrae may be fused together in a similar manner by repeating the foregoing steps of drilling a hole 23 between each pair of adjacent vertebrae and inserting a bone graft dowel 24 therein.

The second form of vertebrae spreader shown in Figures 10-14 of the drawings is a combined drill guide and vertebrae spreader and is used in place of the separate drill guide 3 shown in Figures 1 and 2; the mandrel 10 shown in Figure 3 and the vertebrae spreader shown in Figures 4-7.

The combined vertebrae spreader and drill guide shown in Figures 10-14 is similar to the vertebrae spreader shown in Figures 4-7 with the addition of a collar 40 which is slidable along the outside of the cylindrical tube 11 of the vertebrae spreader between the collar portions 12, 13 and the fixed outer end collar 17. Like parts in the second form of vertebrae spreader which appear in the first vertebrae spreader are shown by the same reference numbers. The purpose of the collar 40 when pushed along the tube 11 to the position shown in Figures 10 and 11 is to hold the two collar portions 12, 13 firmly in their contracted positions, thereby to enable the tube 11 to be used as the drill guide as indicated in Figure 11 in the same way as will the separate drill guide 3. The drill 8 is shown in Figure 11 with a stop 41 thereon which is engageable with the outer end collar 17 to limit the travel of the drill. Figure 10 shows the use of the aforesaid locating mandrel 42 which has a flattened or reduced end which is inserted between the vertebrae 1, 2 and is used to locate the combined vertebrae spreader and drill guide while the pins 16 are driven into the respective vertebrae 1, 2. The mandrel 42 is removed through the tube 11 when the combined vertebrae spreader and drill guide has been correctly positioned on the vertebrae 1, 2 to provide access for the drill 8. The combined vertebrae spreader and drill guide may be removed where the hole is drilled in several stages. Each time the vertebrae spreader is replaced it can be correctly located by inverting the mandrel 42 and inserting its cylindrical end into the partly drilled hole in the same way that the mandrel 10 was used to locate the vertebrae spreader shown in Figure 4.

When the hole in the vertebrae 1, 2 has been drilled, the collar 40 is slid along the tube 11 to abut the outer end collar 17 as shown in Figures 12 and 13. In this position, the collar 40 is engaged by a hook 43 mounted on the collar 17 and is held from sliding back along the tube 11 until the hook 43 has been manually opened to release the collar 40. The collar 40 is formed with diametrically-opposed recesses 44 to provide access through the collar 40 for the expanding rods 19 which are inserted through and screwed into the collar 17 in the same way as described in connection with Figures 4-7. To ensure that the recesses 44 are maintained in alignment with the gaps 15 and the holes for the rods 19, the collar 40 is prevented from turning on the tube 11 by a longitudinally-extending rod 45 extending between the collars 40 and 17 and forming a key along which the collar 40 is slidable. When the collar 40 has been moved into the position shown in Figure 12, the collar portions are freed to enable them to be moved apart and the expanding rods 19 are inserted through the collars 17 and 40 and screwed in to move the collar portions 12, 13 apart to their expanded positions. Then the bone graft dowel 24 is inserted by means of the inserting tool 27, as described with reference to Figures 7 and 8. When the bone graft dowel 24 has been correctly positioned, the collar portions 12, 13 are contracted by unscrewing the rods 19 so that the vertebrae 1, 2 will grip the dowel 24. Then the inserting tool 27 and the vertebrae spreader are removed and the operation is completed, as before.

The combined vertebrae spreader and drill guide shown in Figures 10-14, therefore enables the same operational steps to be taken as described with reference to Figures 1-9, by using the single combined vertebrae spreader and drill guide instead of the separate drill guide 3 of Figure 1 and vertebrae spreader of Figure 4. The combined vertebrae spreader and drill guide may be provided in different bore sizes depending on the diameter of dowel and drill used. The change in bore size may be accommodated by using interchangeable inner sleeves 46 of different bore diameter to be inserted into the tube 11.

Although the vertebrae spreaders and the associated tools and instruments are particularly intended for use in the Cloward method, modified as described herein, of fusing cervical vertebrae together, a similar vertebrae spreader may be suitable with appropriate modification for use for fusing other vertebrae together.

As aforesaid, the use of the vertebrae spreaders provided by this invention enable the dowel to be inserted with less risk of damage to the vertebrae or adjacent vertebrae. Additionally by using the separate drill guide or the combined vertebrae spreader and drill guide, the hole straddling the vertebrae can be accurately made and so the dowel can be made a precise fit in the hole so that it will be gripped tightly and accurately in position when the collar portions 12, 13 of the vertebrae spreader are contracted. Furthermore, the use of the dowel inserting tool which is co-axially aligned with the vertebrae spreader ensures correct alignment of the dowel in the hole.   The dowel can be fitted into the hole without having to hammer the dowel into position, thereby avoiding damage to the dowel or the vertebrae and the accurate fit of the dowel in the hole leads to good fusion and reduces the tendency for post-operative complications to arise.

CLAIMS

1. A vertebrae spreader for spreading apart two adjacent vertebrae to receive a bone graft dowel therebetween, characterised in that the vertebrae spreader comprises a tubular sleeve (11); a pair of substantially semi-cylindrical end portions (12, 13) formed separately from the remainder of the sleeve (11) at one end thereof; a pair of springy blades (14) extending longitudinally of the cylindrical wall of the sleeve (11) and resiliently biased towards each other into circumferentially contracted positions by the resilience of the blades (14) and each carrying a respective one of said end portions (12, 13); adjustable expander means (19) movable into and out of positions between said end portions (12, 13) in which the expander means (19) will force said end portions (12,13) apart laterally of the sleeve (11) from their circumferentially contracted positions against the resilience of the blades (14) and on each said end portion (12, 13) at least a pair of pins (16) extending longitudinally-outwardly therefrom and locatable in respective locating holes (9) which have previously been made in a peripheral wall of each of a pair of vertebrae (1, 2) to be spread apart in their longitudinal direction, the sleeve (11) having a longitudinal throughway of a diameter through which a bone graft dowel (24) is freely insertable.

2. A vertebrae spreader as claimed in Claim 1 in which the blades (14) are formed by parts of the cylindrical wall of the sleeve (11) in the manner of a spring collet.

3. A vertebrae spreader as claimed in Claim 1 or 2 in which the expander means are rods (19) which are adjustable longitudinally of the sleeve (11) and have ends insertable between said end portions (12, 13) of the sleeve (11).

4.    A vertebrae spreader as claimed in Claim 3 in which said ends of the rods (19) which are insertable between said end portions (12, 13) are of non-circular cross-section.

5.    A vertebrae spreader as claimed in Claim 3 or 4 in which the ends of the rods (19) which are insertable between said end portions (12, 13) are tapered in a direction to move said end portions (12, 13) apart when said rods (19) are inserted between said end portions (12, 13).

6.    A vertebrae spreader as claimed in Claims 3-5 in which the rods (19) are adjustable longitudinally of the sleeve by means of screw-threaded portions of the rods (19) extending through respective screw-threaded guides on the sleeve (11).

7.    A vertebrae spreader as claimed in any preceding claim including a locking sleeve (40) which is movable between a first position in which said pair of semi-cylindrical end portions (12, 13) are held by the locking sleeve (40) in positions in which they are contracted, thereby enabling the vertebrae spreader to be used as a drill guide, and a second position in which said pair of semi-cylindrical end portions (12, 13) are free to be moved to their circumferentially expanded positions by said expander means (19). (Figs 10 - 14)

8.    A vertebrae spreader as claimed in Claim 7 including manually-releasable catch means (43) by which the locking sleeve (40) is held in said second position.

9.    The combination of a vertebrae spreader according to any one of Claims 1-6 and a separate tubular drill guide to be used to receive and guide a drill while the latter is being used to drill a hole to straddle a pair of adjacent vertebrae (1, 2), the drill guide (3) having a plurality of pins (4) extending longitudinally from the forward end thereof and which are driven into the peripheral wall of each of the vertebrae (1, 2) to locate the drill guide (3) prior to drilling, the pins (4) on the drill guide (3) producing at least two locating holes (9) in each vertebrae (1, 2), which locating holes (9) are subsequently used, after removal of the drill guide (3), to locate the respective end portions of the vertebrae spreader, the vertebrae spreader (11-13) and the drill guide (3) each having the same number of pins arranged in the same configuration to enable the vertebrae spreader, when the end portions (12, 13) thereof are in their contracted positions, to be located by the pins (19) thereon in the same position as was the drill guide (3) with respect to the vertebrae (1, 2)    (Figs. 1 & 2)

10.    The combination according to Claim 9 and a cylindrical mandrel (10) having a diameter substantially equal to that of the drill to be used to drill the hole which straddles the vertebrae (1, 2), whereby the mandrel (10) is insertable in the hole after the drill and the drill guide (3) have been removed and thus forms a support along which the vertebrae spreader (11-13) is slidable to enable the pins (19) of the vertebrae spreader (11-13) to be located by and driven into the locating holes (9) previously made in the vertebrae (1, 2) by the pins (4) of the drill guide (3), the diameter of the mandrel (10) being smaller than the diameter of the throughway in the sleeve (11) of the vertebrae spreader.    (Figs. 3 & 4)

11.    The combination of a vertebrae spreader according to any one of Claims 1-8 and a tool (28) for inserting said bone graft dowel (24) through the throughway in the sleeve (11) of the vertebrae spreader and into the hole which straddles the vertebrae (1, 2) after the latter have been spread apart by appropriate adjustment of the expander means (19) of the vertebrae spreader to force said end portions (12, 13) thereof and hence the vertebrae (1, 2) apart, the bone graft dowel inserting tool (28) having a cylindrical body (27) which is insertable into and slidable in the throughway in the sleeve (11) of the vertebrae spreader and which has a pin (26) extending forwardly therefrom on which the bone graft dowel (24) is supported as a substantially co-axial forward extension of the body (27) of the inserting tool (28). (Figs. 7, 8 and 13).

12.    The combination according to Claim 11 in which the bone graft dowel inserting tool (28) has an adjustable stop (29) thereon which is locatable against the outer end of the vertebrae spreader to limit the penetration distance of the bone graft dowel (24) into the hole which straddles the vertebrae (1, 2) before the latter are closed together onto the bone graft dowel (28) by adjustment of the expander means (19) of the vertebrae spreader (11-13) to permit the end portions (12, 13) thereof to return to their circumferentially contracted positions.

FIG.1

FIG.2

FIG.3

FIG.4

0077159

2/3

FIG.5

FIG.6

FIG.7

FIG.8

FIG.9

0077159

3/3

FIG.10

FIG.11

FIG.12

FIG.13

FIG.14

European Patent
Office

**EUROPEAN SEARCH REPORT**

Application number

## DOCUMENTS CONSIDERED TO BE RELEVANT

EP 82305253.5

| Category | Citation of document with indication, where appropriate, of relevant passages | Relevant to claim | CLASSIFICATION OF THE APPLICATION (Int. Cl. ³) |
|---|---|---|---|
| A | US - A - 3 486 505 (G.M.MORRISON)<br><br>* Totality *<br><br>-- | | A 61 B 17/00<br>A 61 M 29/00 |
| A | US - A - 3 916 907 (W.C. PETERSON)<br><br>* Totality *<br><br>-- | | |
| A | SURGERY, vol. 32, July-December 1952, St. Louis, USA<br>R.B. CLOWARD "Lumbar inter-vertebral disc surgery"<br>pages 852-857<br>   * Fig. 1-3; page 855 *<br><br>---- | | |

|  | TECHNICAL FIELDS SEARCHED (Int. Cl. ³) |
|---|---|
|  | A 61 B 17/00<br>A 61 M 29/00 |

The present search report has been drawn up for all claims

| Place of search | Date of completion of the search | Examiner |
|---|---|---|
| VIENNA | 20-01-1983 | LUDWIG |